# EUROPEAN PATENT APPLICATION

(11) **EP 1 767 284 A1**
(43) Date of publication of application: **28.03.2007**
(21) Application number: 05739215.1
(22) Date of filing: 10.05.2005
(51) Int. Cl.: B09B 3/00, C01B 3/00, C02F 11/04, C10J 1/00, C12P 3/00, C12P 5/02, C12R 1/145, C12R 1/01

(54) **METHOD OF BIOMASS PROCESSING**

(30) Priority: 18.06.2004 JP 2004180524
(71) Applicant: Hrein Energy, Inc., Hokkaido, 0600002 (JP); MIC, iNC., 14-1, Youkoudai 5-chome, Sagamihara-shi Kanagawa, 2290026 (JP)
(72) Inventor: TAGUCHI, Fumiaki, Sagamihara-shi, Kanagawa 2290026 (JP); SUGAI, Y. c/o DENSEI, INC., 2-1, Techno-park, Sapporo-shi, Hokkaido 0040015 (JP); KUDO, Yasuhiro c/o DENSEI, INC., 2-1, Techno-park, Sapporo-shi, Hokkaido 0040015 (JP); TSURUMI, Rika c/o DENSEI, INC., 2-1, Techno-park, Sapporo-shi, Hokkaido 0040015 (JP); ISHIHARA, S. c/o DENSEI, INC., 2-1, Techno-park, Sapporo-shi, Hokkaido 0040015 (JP)
(74) Representative: Schäfer, Matthias W.
(86) International application number: PCT/JP2005/008502
(87) International publication number: WO 2005/123286

(57) **Abstract**

A novel method of biomass processing, in which while carrying out efficient fermentation of biomass, energy can be recovered without the use of plants and in which after the biomass processing the concentration of organic matter in waste liquid can be reduced. There is provided a method of biomass processing, comprising carrying out a hydrogen fermentation of biomass with the use of a hydrogen producing bacterium to thereby recover hydrogen. Further, a fermentation liquid occurring after the hydrogen fermentation is subjected to methane fermentation with the use of a methane bacterium to thereby recover methane. In this instance, at least an organic acid is contained in the fermentation liquid.

## Description

### Technical Field

The present invention relates to biomass processing. In more detail, it relates to a novel method of biomass processing, in which while carrying out efficient fermentation of biomass, energy can be recovered without using plants and the concentration of organic matter in liquid waste can be reduced after the biomass processing.

### Background Art

A tremendous amount of garbage (biomass) is discharged from a food production process, and use of a garbage treatment apparatus for treating the garbage is well known. In such a garbage treatment apparatus, an aerobic bacterial flora is provided, and the garbage is put into the aerobic bacterial flora so as to be decomposed.

However, a problem lies in that at least 10 wt% or more of the charged garbage cannot be processed and remains as an immature remainder. In addition, for instance, when the immature remainder is used as compost, about another two months of fermentation is required because the immature remainder cannot be used as compost leaving it as it is. Furthermore, if the compost prepared after putting so much work falls in a state of no receiver due to failure in making liaison with farmers or gardeners, it is spoiled and becomes a so-called secondary industrial waste.

The present inventor has proposed a method of treating biomass more efficiently than the previously known method by using, for instance, a hydrogen producing bacteria as a microorganism (for instance, Patent Document 1). In addition, a hydrogen production method for efficiently producing hydrogen from garbage (biomass) which is an organic waste by use of a hydrogen producing bacteria, a methane producing bacteria, and a plant having hydrogen producing ability, is proposed (Patent Document 2).

Patent Document 1 : Japanese Patent Application Laid-open No. 2001-157595 (refer to ABSTRACT, and column number 0035 to 0039)
Patent Document 2 : Japanese Patent Application Laid-open No. 2003-250519 (refer to ABSTRACT, and column number 0054 to 0065)

### Disclosure of the Invention

### Problems to Be Solved by the Invention

The treatment method disclosed in Patent Document 1 is a method of enabling biomass to be efficiently processed and enabling hydrogen to be recovered as clean energy, and research and development toward practical utilization are vigorously conducted. However, a fermented liquid (culture solution) which is liquid waste after recovery of hydrogen contains various organic substances such as hydrogen producing bacteria bodies and acetic acid, butyric acid, or the like as a by-product in high concentration. Such a fermentation liquid containing organic substances in high concentration cannot be discharged into sewerage or a river as it is. Therefore, any countermeasure such as treatment of the fermentation liquid or the like by use of general aeration type waste water treatment method is required.

The method of producing hydrogen disclosed in Patent Document 2 is to produce hydrogen by giving a substance decomposed by hydrogen fermentation caused by hydrogen producing bacteria, methane fermentation caused by methane bacteria, or the like, as a raw material of hydrogen for a plant having hydrogen producing ability. Accordingly, other than hydrogen producing bacteria and methane bacteria, utilization of a plant which is complicated in the method of nurture (cultivation method) is required, which is time-consuming.

Furthermore, in recent years, since environmental preservation and effective use of sources (recycling) have attracted considerable attention, food related waste and/or living related waste among biomass are/is needed to be processed more efficiently. A method to effectively recycle highly concentrated organic matter in a fermentation liquid generated after microbial treatment of biomass composed of such a food related waste and/or a living related waste and to clean up the fermentation liquid to a degree that the fermented liquid (liquid waste) can be discharged into sewerage or a river without anxiety is demanded. However, such a report or the like has not been submitted yet at the present time.

The invention in this application is achieved based on the above-described background, and the object thereof is to provide a novel method of biomass processing, which solves conventional problems, can efficiently perform fermentation treatment of biomass, can recover energy without using a plant, and can lower the concentration of organic matter contained in liquid waste after biomass processing.

### Means to Solve the Problems

In order to solve the above-described problems, the present invention performs hydrogen fermentation process of biomass by using a hydrogen producing bacteria which contains at least Clostridium beijerinkii AM 21 B strains, Clostridium sp. No. 2 strains, and Clostridium sp. X 53 strains to recover hydrogen, and performs methane fermentation treatment of the fermentation liquid generated after the hydrogen fermentation process by using methane bacteria to recover methane. The fermentation liquid must contain any organic acid.

When structured as above, fermentation treatment, namely hydrogen fermentation process can be conducted by affecting biomass on the hydrogen producing bacteria so that hydrogen is produced through this hydrogen fermentation process. In particular, since this treatment is hydrogen fermentation process by using hydrogen producing bacteria containing at least one of Clostridium beijerinkii AM 21 B strain, Clostridium sp. No.2 strain, and Clostridium sp. X53 strain, high hydrogen yield can be expected compared with the cases of performing hydrogen fermentation process using other methods or other hydrogen producing bacteria.

In addition, the fermented liquid which is waste water occurred after the hydrogen fermentation can be processed by methane fermentation treatment using methane bacteria which have fermenting capability different from the hydrogen producing bacteria so that methane can be produced by this methane fermentation treatment. In such cases, it becomes possible to reduce the concentration of the organic matter contained in the liquid waste after the biomass processing, because the methane fermentation is conducted using fermented liquid after hydrogen fermentation. Accordingly, it becomes possible to discharge the fermented liquid (liquid waste) into sewerage or a river at ease after methane fermentation.

Furthermore, since organic acid is contained in a fermentation liquid, methane fermentation by methane bacteria can be efficiently performed owing to the existence of such an organic acid. In addition, since use of plants having hydrogen production capability is not required, it is possible to further reduce the costs without expense in time and effort for nurture (cultivation method).

Further, in other inventions in addition to the above-described invention, food related waste and/or living related waste are/is contained in biomass. Accordingly, it can realize effective use of resources, and at the same time, since it can prevent food related waste and/or living related waste from being disposed as wastes, environmental preservation can be realized.

Further, in another invention in addition to the above-described invention, hydrogen recovered by the hydrogen fermentation is formed in a liquid by compression process. When structured like this, it makes transportation and storage of hydrogen are facilitated. In addition, since it needs only compression process, it can reduce the costs for transportation and storage.

Further, in another invention in addition to the above-described invention, hydrogen recovered by hydrogen fermentation is subject to occlusion in a carbon nanotube. When structured in this manner, it is possible to occlude an extremely great amount of hydrogen on a weight percentage basis by the carbon nanotube. Accordingly, it becomes possible to store a greater amount of hydrogen than the case of compressing hydrogen in a vessel or the like. It is also possible to avoid danger of explosion because there is no compression process.

Further, in another invention in addition to the above-described invention, hydrogen recovered by hydrogen fermentation is subjected to occlusion by a hydrogen occlusion alloy. When composing the structure of biomass processing in this manner, it is possible to occlude a greater amount of hydrogen on a weight percentage basis by the carbon nanotube. Accordingly, it becomes possible to store a greater amount of hydrogen than the case of compressing hydrogen in a vessel or the like. It is also possible to avoid danger of explosion because there is no compressive treatment.

Further, in another invention in addition to the respective inventions described above, methane recovered by methane fermentation is formed in a liquid form by compression. When composing in this manner, it makes transportation and storage of methane easy. In addition, since it needs only compression process, it can reduce the costs for transportation and storage.

Furthermore, another invention, in addition to the respective inventions described above, performs methane fermentation using at least one kind of bacteria belonging to the genus Methanobacterium, the genus Methanococcus, the genus Methanosarcina, the genus Methanosaeta, and the genus Methanohalophillus.

When structured in this manner, the methane fermentation is to be performed using methane bacteria having excellent properties, which results in large amount of methane production.

### Effect of the Invention

According to the present invention, it is made possible to effectively perform fermentation treatment of biomass. In addition, it can recover energy without using plants. Furthermore, it becomes possible to reduce the concentration of organic matter contained in liquid waste after biomass processing.

### Brief Description of Drawings

Fig. 1 is a side cross sectional view showing a structure of a hydrogen producing apparatus relating to a second embodiment of the present invention;
Fig. 2 is a flow chart showing a hydrogen producing method relating to the second embodiment of the present invention;
Fig. 3 is a view showing a relational example of the reaction time and the amount of hydrogen produced when an organic material and a Clostridium genus microorganism are reacted using the hydrogen producing apparatus in Fig. 1;
Fig. 4 is a side cross sectional view showing a configuration of a hydrogen producing apparatus relating to a third embodiment of the present invention;
Fig. 5 is a flow chart showing a hydrogen producing method relating to the third embodiment of the present invention;
Fig. 6 is a side cross sectional view showing a hydrogen/methane producing method relating to a fourth embodiment of the present invention; and
Fig. 7 is a flow chart showing a hydrogen producing method relating to the fourth embodiment of the present invention.

### Explanation of Codes

- 10, 30, 40: hydrogen producing apparatus

- 11: reaction vessel
- 12, 32: piping member
- 12a, 32a: material charging port
- 13: microorganism supply pipeline
- 14, 23: regulating valve
- 15: fin (a portion of a first agitation means)
- 16: motor (a portion of the first agitation means)
- 17: monitor (control means)
- 20: microorganism pre-cultivation bath (a form of microorganism pre-cultivating means)
- 21: culture solution tank (a form of culture solution storing means)
- 22: culture solution supply pipeline
- 24: hydrogen discharge pipeline
- 31: sterilization mechanism
- 33: boiler unit
- 33a: space portion
- 35: water charging pipe (a form of liquid charging means)
- 35a: water charging port
- 36: heating mechanism
- 37: pressure adjusting mechanism
- 41: methane producing mechanism
- 43: solid-liquid separation bath (a form of solid-liquid separation means)
- 44: solid final treatment bath (a form of the final solid treatment means)
- 46: fin (a portion of a second agitation means)
- 47: motor (a portion of the second agitation means)
- 49: methane fermentation bath (methane fermentation means)
- 50: fin (a portion of a third agitation means)
- 51: motor (a portion of the third agitation means)

### Best Mode for Carrying out the Invention

### (First Embodiment)

Hereinafter, a method of biomass processing relating to the first embodiment of the present invention will be explained in detail. However, the invention in this application should not be limited by the following embodiments.

The method of biomass processing according to the invention in this application performs fermentation treatment, namely, hydrogen fermentation process by acting biomass such as food related waste, living related waste and the like upon hydrogen producing bacteria (first step). Hydrogen is produced by the hydrogen fermentation process, the hydrogen can be efficiently recovered, and at the same time, the biomass can be efficiently decomposed.

Then, methane fermentation of a fermented liquid which is liquid waste occurred after the hydrogen fermentation is conducted (second step), using a microorganism having fermentation treatment capability different from the hydrogen producing bacteria, namely, methane bacteria. At this time, a fermented liquid generated by the hydrogen fermentation process being the first step may be introduced to methane fermentation treatment being the second step which is succeeding fermentation treatment without addition of any action. Needless to say, appropriate treatment such as filtration may be conducted to remove unnecessary organic acid or the like in this methane fermentation. The method of biomass processing according to the invention in this application can also efficiently recover methane produced by such a methane fermentation.

The method of biomass processing method according to the invention in this application having such characteristics can recover various energy (hydrogen and methane) without utilizing plants which require complicated labors for nurture (cultivation method) and have hydrogen producing capability, while efficiently conducting fermentation and decomposition of biomass such as animal waste, plant waste or the like. As described above, since it does not require utilization of the plant having hydrogen producing capability, it becomes possible to further reduce costs without taking labor for methods of nurture (cultivation method) or the like. In addition, it is possible to reduce the concentration of highly concentrated organic matter in a fermented liquid (liquid waste) after hydrogen fermentation. Accordingly, it becomes possible to discharge a fermented liquid (liquid waste) into sewerage or a river at ease.

As described above, it is preferable for the biomass in the method of biomass processing according to the invention in this application that it contains at least food related waste or living related waste, or both of them in terms of environmental preservation, effective usage of resources (recycle), or the like. When food related waste and/ or living related waste are/is contained in biomass, it is possible to realize effective usage of resources which would be thrown away as waste in the present circumstances. Furthermore, since disposal of food related waste and/or living related waste as waste dump is prevented, it can serve as environmental preservation.

The food related waste and the living related waste will be explained in detail. They are animal waste such as chicken, pork, fish waste and the like, plant waste such as bean curd refuse, wheat bran, rice bran, bread or pasta to be disposed including raw flour kneaded with water, vegetable, fruit juice, pomace, draff of soy source or shochu, and the like. Needless to say, it does not matter whether the amount of the waste (garbage) is a level disposed from a household or a level disposed from businesses such as restaurants or the like. The method of biomass processing according to the invention in this application is able to be used satisfactory on any scale needed.

The methane fermentation using methane bacteria can be efficiently conducted on condition that the fermentation liquid contains at least an organic acid. This organic acid is organic acid useful for methane fermentation in the methane fermentation treatment being the second step (corresponding to fermentation stroma). As a concrete example, various organic acids such as acetic acid, butyric acid, phosphoric acid, citric acid, pyruvic acid, malic acid, succinic acid, lactic acid, formic acid, levulinic acid, or the like can be cited as examples, and it is preferable that at least one kind or more of these examples are contained in a fermentation liquid as an organic acid. It should be noted that even if the fermentation liquid contains fungus bodies of hydrogen producing bacteria or the like, it is considered to have no problem because it interferes little with methane fermentation treatment. As for organic acid, the organic acid produced by hydrogen fermentation can be used, and it is also possible to add organic acid when the amount necessary for the methane fermentation is insufficient.

Hydrogen recovered by the method of biomass processing according to the invention in this application has characteristics of being environmentally friendly energy (clean energy) and high in energy conversion efficiency because it does not contain carbon and does not produce carbon dioxide on burning. In particular, since the by-product produced after usage (burning) is water, this method of biomass processing is environmentally friendly compared with fossil fuel such as conventional petroleum or the like. In addition, hydrogen has combustion energy larger than petroleum by about three times as much as petroleum. Hydrogen producing bacteria can efficiently produce hydrogen excellent in terms of such environmental aspect and efficiency. Methane produced from a fermented liquid (liquid waste) produced after hydrogen fermentation can be applied widely in various fields, and can be used as an energy source for various machines and apparatuses. Methane bacteria can efficiently produce such kind of methane.

It should be noted that forms of energy, hydrogen, and methane recovered may be gas, liquid, or a solid. Further, the form can be appropriately processed or converted to a form easily applicable as energy (fuel). For instance, it is possible that the energy recovered as a gas (hydrogen gas, methane gas) can easily be converted into liquid by compression. In such cases, it becomes easy to transport or store hydrogen and methane. Furthermore, since compression is all that required, it can reduce costs for transportation and storage.

Furthermore, hydrogen may be stored by being absorbed in a carbon nanotube. When such a carbon nanotube is used, a great deal of hydrogen in weight ratio can be occluded by the carbon nanotube. Therefore, it is possible to store much greater amount of hydrogen than in the case of compression process and storing it into a container or the like. Since it is not compressed or the like, it is also possible to prevent the danger of explosion. As such a carbon nanotube, single-walled carbon nanotubes (SWNT) and graphite nanofibers (GNF) are suitable. In this case, hydrogen absorption may be performed at low temperatures/under pressurized pressure or at room temperatures/under normal pressure. In addition, such carbon nanotube may be produced using methane produced by methane fermentation, which will be described later, as a raw material. It is also possible to add potassium (K), lithium (Li), or the like to the carbon nanotube produced.

Furthermore, it is possible that hydrogen is stored by being absorbed by a hydrogen occlusion alloy. When such a hydrogen occlusion alloy is used, it is possible to occlude a great deal of hydrogen in weight ratio by the carbon nanotubes. Accordingly, a greater deal of hydrogen than the amount in the case of compressing it into a container or the like can be stored. Since it is not compressed or the like, it is also possible to prevent a danger of explosion. As such a hydrogen occlusion alloy, for instance, a magnesium series alloy such as MgH₂, a titanium series alloy such as FeTiH₂, and a vanadium series alloy such as Ti-V-Mn, Ti-V-Cr can be cited.

Furthermore, hydrogen producing bacteria belonging to genus Clostridium separated by the present inventor such as Clostridium beijerinkii AM 21 B strain (refer to Journal of Fermentation and Bioengineering 73 :244 to 245, 1992), Clostridium sp. No. 2 strain (refer to Canadian Journal of Microbiology 40:228 to 233, 1994), and Clostridium sp. X53 strain (refer to Journal of Fermentation and Bioengineering 81:178 to 180, 1996) are cited as hydrogen producing bacteria in the method of biomass processing according to the invention in this application. However, various hydrogen producing bacteria can be used as the hydrogen producing bacteria so far as they can produce hydrogen while efficiently treating biomass, and are not limited to the above-described hydrogen producing bacteria.

When using such hydrogen producing bacteria, the hydrogen fermentation is performed by use of the hydrogen producing bacteria provided with excellent property in hydrogen production, the amount of hydrogen produced can be made greater compared with the case of performing hydrogen fermentation process by other methods.

As the methane bacteria, there are bacteria belonging to such as genus Methanobacterium, genus Methanococcus, genus Methanosarcina, genus Methanosaeta), and genus Methanohalophillus. However, various methane bacteria can be used as the methane bacteria so far as they can efficiently generate methane while treating biomass and fermented liquid after hydrogen fermentation process, without being limited to the above-described methane bacteria.

When using such methane bacteria, it means that the methane fermentation is performed by use of the methane bacteria having excellent property in methane production, so that a big amount of methane can be produced.

The invention in this application having the above-described characteristics will be explained in more detail, and concretely. It is needless to say that the invention in the application is not limited by the following examples.

### Example

### 1. Hydrogen Fermentation Process

### (1) Pre-cultivation of Hydrogen Producing Bacteria

Hydrogen producing bacteria were inoculated in 100 ml of a PY culture medium in a 300 ml Erlenmeyer flask to which 0.1% glucose is added, and cultivation over night was carried out in an anaerobic glove box (manufactured by US Former Co., Type 1024) at 37°C. The composition of the PY culture medium is 10g peptone, 5g yeast extract, 500 mg L-cystein HCl, 8mg CaCl, 8 mg MgSO₄, 40 mg KH₂PO₄, 400 mg NaHCO₃ and 80 mg NaCL, and no carbon source.

### (2) Hydrogen Fermentation Method

1400 ml of the PY culture medium containing 7.5g of starch or glucose serving as a hydrogen production source, and 100 ml of pre-cultivation bacteria solution for hydrogen producing bacteria were charged to a 3000 ml Erlenmeyer flask, and after a rubber stopper provided with a gas discharge port, a insertion port of a pH controller (manufactured by Tokyo Rikaki, Type FC-10) and an NaOH solution inlet port for pH adjustment and the like was stuffed, it was put outside the anaerobic glove box. It was kept in a constant temperature water bath at 37°C, and agitated with a magnetic stirrer.

### (3) Recovery of Hydrogen and Quantitative Method

The generated gas was recovered in a measuring cylinder by a water displacement method after the gas was passed through 10% NaOH solution to remove carbon dioxide and other NaOH solution soluble gases, and determined the quantity. The recovered gas was confirmed to be hydrogen gas from the result by a high speed gas chromatography analysis and the sound of explosion during a burning test.

The fermented liquid (liquid waste) generated by the hydrogen fermentation process was used for the succeeding methane fermentation treatment. At this time, the fermented liquid (liquid waste) may be the one without treatment, or may be the one for which various treatment such as filteration was performed.

### II. Methane Fermentation Treatment

### (1) Methane Fermentation Treatment Apparatus

The methane fermentation treatment was conducted by the upflow anaerobic sludge blanket (UASB) method. The treatment apparatus used for the experiment was a cylindrical one having an interior content of 10 L with a depth of 930 mm, attached with a stirrer which can gently stir at the bottom of the apparatus. An inlet port is provided at the bottom of the treatment apparatus and an overflow dam is provided at the top thereof, so that a generated gas can be taken out from the top of the treatment apparatus.

The above-described fermented liquid to perform methane fermentation treatment is fed into the inlet port by a metering pump. The liquid flowing out from the overflow dam was recovered as a treated liquid (final liquid waste). A generated gas was measured with a gas metering device. A water jacket was provided to the methane fermentation treatment apparatus to keep it at 35°C.

### (2) Experimental Conditions

7.6L of returned sludge from the living waste water treatment facility was poured into the methane fermentation apparatus, a fermented liquid (liquid waste) after hydrogen fermentation was poured in from the inlet port with a metering pump, and methane fermentation treatment was started. The amount of inflow of the fermented liquid (liquid waste) was set to be 0.3 L/day or 1 L/day, and the fermentation liquid was intermittently poured-in by operating a metering pump two times a day, for two hours at a time. The treatment was conducted continuously for about five months after the treatment was started. During this period, since the amount of the generated gas was confirmed to be almost steady after 15 days, the generated gas was measured almost every day after this point of time. After three months had passed, the treated water (final liquid waste) was collected about every other week to be analyzed.

### (3) Analysis Items and Analysis Method

In order to determine the effect of the methane fermentation treatment, the biochemical oxygen demand (BOD), the chemical oxygen demand (COD), the total organic carbon (TOC), the inorganic carbon (IC), the Kjeldahl nitrogen (kje-N), the total phosphorous (T-P), the total solid (TS), the volatile materials (VM), the dissoluble materials (DM), the suspended solid (SS), the volatile suspended solid (VSS), the alkalinity, pH and organic acid in the fermented liquid (liquid waste) after hydrogen fermentation process and the treated water (final liquid waste) after methane fermentation treatment were measured respectively. The measurement was conducted according to a conventional method of sewage test. As the COD, the amount of oxygen consumption by potassium permanganate at 100°C under acidic condition was determined. For measurement of TOC and IC, a TOC meter 5000A (manufactured by SHIMAZU Corporation) was used, and for the measurement of organic acid, a gas-chromatography GC-14B (manufactured by SHIMAZU Corporation) was used.

The criterion measure for water discharge of rivers are BOD 120, SS 150 mg/L or less, and the criterion measure for sewage discharge are BOD 600 and SS 600 mg/L or less.

### Embodiment 1: Recovery of hydrogen and methane from biomass (starch)

### (1) Hydrogen Fermentation Process of Starch

1400 ml of the PY culture medium containing 7.5g of starch, and 100 ml of the pre-cultivation bacteria solution of hydrogen producing bacteria (Clostridium beijerinkii AM 21 B strain) were put into a 3000 ml Erlenmeyer flask, and cultivation was started in a constant temperature water bath at 37°C. After two or three hours elapsed from start of keeping the temperature, foaming was observed, and the amount of generated gas reached it's zenith about 7 to 8 hours later. The generation of gas was stopped after 10-odd hours. The amount of hydrogen gas produced was 3750 ml in total. The fermented liquid (liquid waste) which is a cultivation solution after hydrogen fermentation was poured into a methane fermentation bath with no treatment.

### (2) Methane Fermentation Treatment from the Fermentation Liquid of Starch

The fermented liquid (liquid waste) after hydrogen fermentation was flowed into a cylindrical apparatus for methane fermentation treatment based on the UASB method having interior content of 10 L, and the treatment was started. The amount of inflow was set to be 0.3 L/day, and a metering pump was operated 2 times a day, and for two hours per one time to allow the liquid to intermittently flow in. Accordingly, the residence time in the treatment apparatus was 33 days.

The TOC of the treatment water (final liquid waste) after the methane fermentation was about 360 mg/L, the BOD thereof was about 530 mg/L, and the COD thereof was 490 mg/L. The removal rates were 95%, 97%, and 90% respectively (refer to Table 1). The amount of methane gas produced was 1.54L a day. The fermented liquid (liquid waste) after the hydrogen fermentation using this starch was confirmed to be decomposable by anaerobes.

### (3) Properties of Water Quality

The water quality of the fermented liquid (liquid waste) generated by the hydrogen fermentation process described above (1) and that of the treated water (final liquid waste) generated by the methane fermentation treatment described above (2) are shown in Table 1 in a comparison form. As shown in Table (1), it is confirmed that by performing methane fermentation treatment to the fermented liquid (liquid waste) generated in the hydrogen fermentation process, hydrogen gas and methane gas can be recovered as described above, TOC, BOD, COD and the like can be effectively removed. Accordingly, since the final liquid waste has lower values than the criterion measure for sewage discharge, labor for the treatment can be reduced.

**Table 1**

| Item | Liquid waste after hydrogen ferment. average value (mg/L) | Liquid waste after methane ferment. average value (mg/L) | Removal rate |
|---|---|---|---|
| TOC | 6,920 | 357 | 95% |
| BOD | 17,800 | 530 | 97% |
| COD | 4,690 | 490 | 90% |
| TS | 16,077 | 3,202 | 80% |
| VM | 14,305 | 1,248 | 91% |
| IM | 15,064 | 3,113 | 80% |
| SS | 644 | 90 | 86% |
| VSS | 596 | 77 | 87% |
| Kie-N | 1,872 | 1,508 | 19% |
| T-P | 207 | 190 | 8% |
| pH | 4.89 | 8.48 | - |

| | | | |
|---|---|---|---|
| TOC : total organic carbon, TS : total solid, VM : volatile matter, DM : dissolved matter, SS : suspended solid, VSS : volatile suspended solid, kje-N : kjeldahl nitrogen, T-P : total phosphorous | | | |

### Example 2 : Recovery of Hydrogen and Methane from Biomass (Glucose)

### (1) Hydrogen Fermentation Process of Glucose

1400 ml of culture solution prepared by dissolving glucose 7.5g into a diluted PY culture medium made by diluting a PY culture medium with five times of ion-exchange water, and 100 ml of pre-cultivation bacteria solution of hydrogen producing bacteria (Clostridium beijerinkii AM 21B strain) are put into a 3000 ml Erlenmeyer flask, and cultivation was started in a constant temperature water bath at 37°C. After two hours elapsed from start of keeping temperature, foaming occurred, and the amount of generated gas reached it's zenith about 7 hours later. The generation of gas was stopped after 10-odd hours. The amount of generated hydrogen gas was 5750 ml in total. The fermented liquid (liquid waste) after hydrogen fermentation was poured into a methane fermentation bath without treatment.

### (2) Methane Fermentation Treatment from Fermentation Liquid of Glucose

The amount of the fermented liquid (liquid waste) poured after the hydrogen fermentation is set to be 1 L/day, and a metering pump is operated twice a day, for 2 hours at each time so that the liquid is intermittently flowed in. Accordingly, the residence time was for 10 days.

The TOC of the treated water (final liquid waste) after the methane fermentation was 48 mg/L, the BOD was 10 mg/L, and the COD was 71 mg/L. Each removal rate was 99%, not less than 99%, and 98% respectively. The amount of producing methane gas was 4.85L per day. The fermented liquid (liquid waste) after the hydrogen fermentation using the diluted PY culture medium was able to be confirmed to be decomposable by anaerobes.

### (3) Properties of Water Quality

The water quality of the fermented liquid (liquid waste) generated by the hydrogen fermentation process described above (1) and that of the treated water (final liquid waste) generated by the methane fermentation treatment described above (2) are shown in Table 2 in a comparison form. As shown in Table (2), it is confirmed that by performing methane fermentation treatment to the fermented liquid (liquid waste) generated in the hydrogen fermentation process, hydrogen gas and methane gas can be recovered as described above, TOC, BOD, COD and the like can be effectively removed. Accordingly, since the final liquid waste had lower values than the criterion measure for river and sewage discharge, labor for the treatment of the final liquid waste was able to be reduced.

**Table 2**

| Item | Liquid waste after hydrogen ferment. (mg/L) | Liquid waste after methane ferment. (mg/L) | Removal rate |
|---|---|---|---|
| TOC | 4,763 | 48 | 99% |
| BOD | 12,274 | 10 | >99% |
| COD | 4,264 | 71 | 98% |
| SS | 1,055 | 140 | 87% |
| PH | 4.21 | 8.2 | - |

### (Second Embodiment)

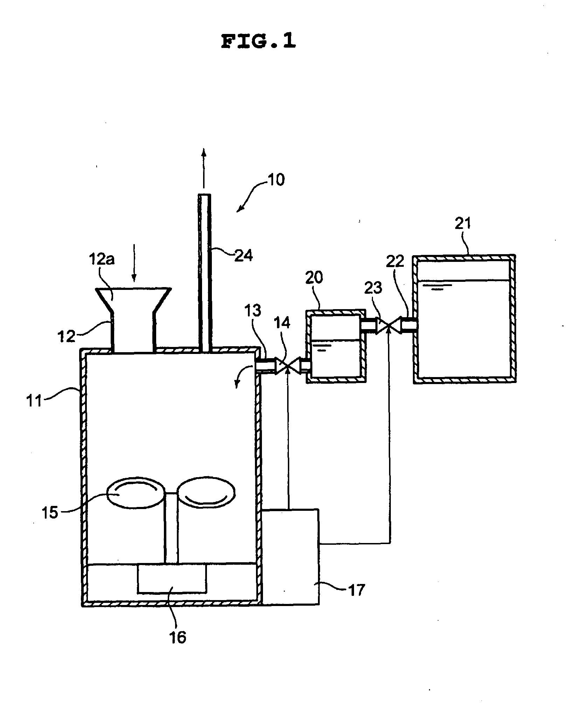

Hereinafter, the second embodiment of the present invention will be explained referring to Figs. 1 to 3. Fig. 1 is a side cross sectional view showing a configuration of a hydrogen producing apparatus 10 used in the present embodiment. In the drawing, the hydrogen producing apparatus 10 includes a reaction vessel 11. A material charging port 12a to charge materials for hydrogen production is provided in the reaction vessel 11. Though not shown, when the material to be charged is a solid, a pulverizer is provided at a portion of the material charging port 12a. In the present embodiment, the material charging port 12a with an opening upwards is formed at the upper end of a cylindrical member 12 having a large diameter. The cylindrical member 12 is connected to the upper end face of the reaction vessel 11. Organic matter can be thrown into the inside of the reaction vessel 11 from the cylindrical member 12.

It should be noted that the material charging port 12a may be closed with a lid (not shown) after charge of the material into the inside of the reaction vessel 11. Structured in this manner, it is possible to prevent saprophytic bacteria from entering it from outside.

One end of a microorganism supply pipeline 13 to charge microorganisms (in the present embodiment, a genus Clostridium microorganism ; hereinafter, referred to as a microorganism A) to produce hydrogen as will be described later, is connected to the upper side on the side surface of the reaction vessel 11. Since the other end of the microorganism supply pipeline 13 is connected to a microorganism pre-cultivation bath 20 to be described later, it is possible to supply an appropriate amount of culture solution to the inside of the reaction vessel 11. It should be noted that the culture solution after multiplication of the microorganism A will be explained as a multiplied culture solution. In addition, the culture solution existing in the culture solution tank 21, or the culture solution in a microorganism pre-cultivation bath 20 before multiplication of the miroorganism A will be explained as a culture solution for multiplication.

A regulating valve 14 is provided at a prescribed position of the microorganism supply pipeline 13. By control of opening and closing of the regulating valve 14, and the degree of opening, it becomes possible to regulate/control the amount of supply of the culture solution from a microorganism pre-cultivation bath 20 which will be described later to the reaction vessel 11, and whether the supply is performed or not.

It should be noted that the regulating valve 14 may be provided at the border of the microorganism pre-cultivation bath 20 with the microorganism supply pipeline 13, and the border between the reaction vessel 11 and the microorganism supply pipeline 13, instead of providing the regulating valve 14 inside the microorganism supply pipeline 13.

A suction means (not shown) such as a vacuum pump or the like is connected to the reaction vessel 11 so that the inside thereof can be set under vacuum. A fin 15 is provided as a first agitation means in the inside of the reaction vessel 11. The fin 15 agitates organic matter thrown into the inside of the reaction vessel 11, so as to serve to promote the reaction. As an example of such a fin 15, one vane of the fin having, for instance, two pieces of vanes is provided so as to tilt diagonally upward from the center line, and the other vane is provided so as to tilt diagonally downward from the center line so that agitation is conducted uniformly in every direction.

However, the fin 15 can take any size and shape so far as it can satisfactory agitate the inside of the reaction vessel 11. Furthermore, though the fin 15 is made of stainless steel in the present embodiment, a porous absorptive member such as magnetic materials or ceramics or the like may be used as the whole material. When such a porous material is used, if metal such as mercury or toner for copying exists in organic matter to be put in the reaction vessel 11, it can be recovered without being diffused. Note that agitation can be conducted by, for instance, swinging the whole reaction vessel 11 itself, or rotating the whole reaction vessel 11 instead of agitation with the fin 15.

In addition, a motor 16 directly connecting to the above-described fin 15 is provided to the reaction vessel 11, and agitation is conducted by driving the motor 16. Further, a monitor 17 is provided outside the reaction vessel 11 for monitoring the reaction inside the reaction vessel 11. With the monitor 17, the reaction time, temperature, and pH during the progress of the reaction are monitored, and the reaction time is always calculated. The monitor 17 can inform of the completion of the reaction using information means such as a buzzer, a lamp or the like to the outside.

The monitor 17 serves a function as a controller. That is, the regulating valves 14 and 23 of the microorganism supply pipeline 13 and the culture solution supply pipeline 22, which will be described later, are connected to the monitor 17. Supply of the culture solution from a culture solution tank 21 to be described later to a microorganism pre-cultivation bath 20, and supply of a culture solution containing microorganisms A to the inside of the reaction vessel 11 are controlled by opening control of the regulating valves 14 and 23 at the monitor 17.

A controller to control the control means can be provided separately, instead of letting the monitor 17 serve a function as a controlling means.

The microorganism pre-cultivation bath 20 as a microorganism pre-cultivating means is connected to the other side of the microorganism supply pipeline 13. The microorganism pre-cultivation bath 20 is for multiplication of the microorganism A in the culture solution for pre-cultivation. Further, the microorganism pre-cultivation bath 20 is for maintaining a state that the microorganism A lives in a multiplied culture solution in which multiplication of the microorganism A has been conducted. For this purpose, a temperature adjusting means (not shown) is provided on the microorganism pre-cultivation bath 20. The microorganism pre-cultivation bath 20 is kept at a temperature best suited for multiplication by the function of the temperature adjusting means.

Though about 37 degrees is the most desirable temperature for the multiplication, multiplication of the microorganism A can be sufficiently conducted at temperatures in the range from 25° to 45°, similarly to the reaction progress inside the reaction vessel 11, which will be described later. A temperature other than this range can be adoptable so far as the multiplication can be realized. Various devices such as an electric heater, a heating boiler, or the like can be used as the temperature adjusting means.

One side of the culture solution supply pipeline 22 is connected to the microorganism pre-cultivation bath 20. The other side of the culture solution supply pipeline 22 is connected to the culture solution tank 21 as a culture solution storage means. The culture solution for multiplication suitable to multiply the microorganism A is stored in the culture solution tank 21. A new culture solution for multiplication can be supplied to the microorganism pre-cultivation bath 20 from the culture solution tank 21 after the microorganism A is supplied from the microorganism pre-cultivation bath 20 to the inside of the reaction vessel 11.

A regulation valve 23 is also provided to the culture solution supply pipeline 22. Opening and closing of the regulating valve 23 is controlled by control of the regulating valve 23 with the monitor 17, and is controlled in a suitable amount of opening when it is opened. It should be noted that the regulating valve 23 can be provided at the border between the microorganism pre-cultivation bath 20 and the culture solution supply pipeline 22, or at the border between the culture solution tank 21 and the culture solution supply pipeline 22.

An end of a hydrogen discharge pipeline 24 is connected to the upper end surface of the reaction vessel 11. The hydrogen discharge pipeline 24 is for discharging hydrogen which is low in specific gravity from the reaction vessel 11 to the outside. The other end of the hydrogen discharge pipeline 24 is stored in a hydrogen occlusion alloy not shown or in a hydrogen storage unit (not shown) such as a gas cylinder or the like, which are provided outside. Owing to such a structure, hydrogen is produced from organic matter using the microorganism A, and the produced hydrogen is stored in the hydrogen storage unit.

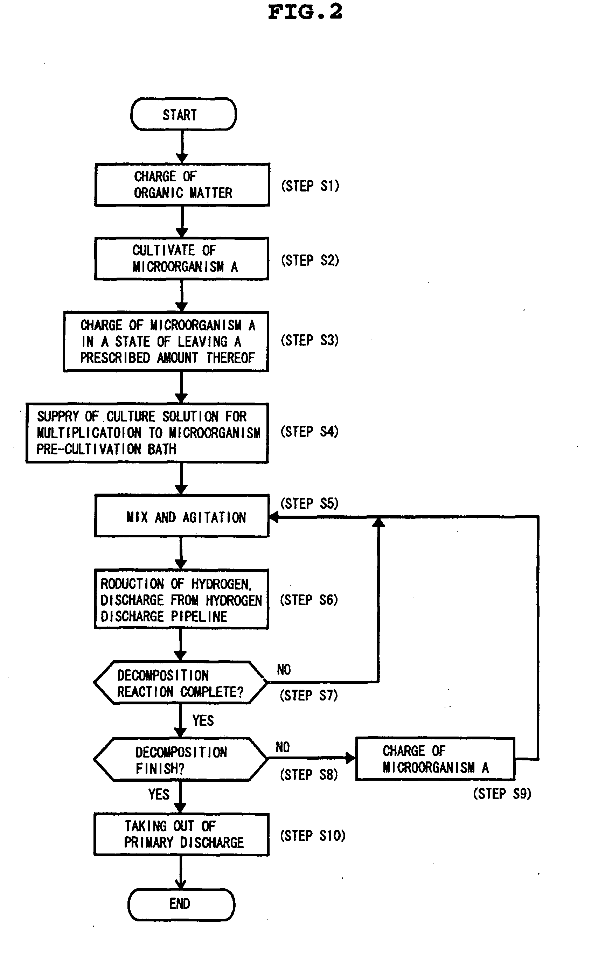

A method of producing hydrogen using the hydrogen producing apparatus 10 as above will be explained hereinafter based on Fig. 2.

First, organic matter is charged inside the reaction vessel 11 (step S 1 ; corresponding to an organic material charge process). The charged organic matter is, for instance, a mixture of a starch material such as potatoes or the like and green color vegetables represented by a cabbage, food grain represented by a core of corn, mid-gut gland which is one of the waste of scallop, the internal organs of livestock, or the like. However, the above-described organic matter is only examples, and a plant organic material such as plant waste or an animal organic material such as animal waste or the like can be used other than the materials described above.

When a starch material is decomposed, though the decomposition reaction can be progressed singly, if organic materials different in nature due to difference in composition are mixed, like a starch material, and green vegetables, mid-gut gland of scallop and the like, these green vegetables or mid-gut gland of scallop serve as enzymes, which results in promotion of the reaction. As above, combination of materials different in kind, not that of materials equivalent in kind is preferable in promotion of reaction.

In advance to the charge process, it is necessary to cultivate the microorganism A in advance in the microorganism pre-cultivation bath 20 (step 2 ; microorganism pre-cultivation process). Accordingly, a culture solution for multiplication is stored in the microorganism pre-cultivation bath 20 first, and the strain of the microorganism A are added to the culture solution for multiplication. The microorganism pre-cultivation bath 20 is set at a prescribed temperature (in the range from 25 degrees to 45 degrees, preferably 37 degrees). It is kept for a certain hour while keeping at this temperature. If the period in which the microorganism pre-cultivation bath 20 is left to stand for the multiplication is between 12 hours and 24 hours, it is preferable because the microorganism A sufficiently multiples. However, any period other than the above-described period is adoptable so far as the multiplication of the microorganism A sufficiently progresses.

After, or in advance to, or concurrently with charging of these organic matter, the genus Clostridium microorganism (microorganism A) being anaerobic bacteria are charged in the reaction vessel 11 from the microorganism pre-cultivation bath 20 via the microorganism supply pipeline 13 in a state of being contained in a multiplied culture solution (step S3). As a genus Clostridium microorganism (microorganism A), for instance, Clostridium beijerinkii AM21B strain (document; Journal of Fermentation and Bioengineering 73:244-245, 1992), genus Clostridium sp. No. 2 strain (document; Canadian Journal of Microbiology 40:228-233, 1994), or genus Clostridium sp. X53 strain (document; Journal of Fermentation and Bioengineering 81:178-180, 1996), etc. can be cited. However, the genus Clostridium microorganism (microorganism A) is not limited to them, and other various strains can be used. It is also possible to use a hydrogen producing microorganism other than genus Clostridium (for instance ; the microorganism other than genus Clostridium in the first embodiment) as the microorganism A.

The multiplied culture solution is charged in the inside of the reaction vessel 11 after the above-described standing time elapses and the microorganism A is sufficiently multiplied (step S3 ; corresponding to supply execution process, and the amount of supply control process). Thereby, the decomposition reaction of organic matter by the microorganism A is started.

When the culture solution is supplied to the inside of the reaction vessel 11, all of the multiplied culture solution in the microorganism pre-cultivation bath 20 is not supplied to the reaction vessel 11, but a prescribed amount of the multiplied culture solution is left in the microorganism pre-cultivation bath 20 by control of the monitor 17 (this part in step 3 corresponds to the amount of supply control process).

Then, the culture solution for multiplication is newly supplied from the culture solution tank 21 to the microorganism pre-cultivation bath 20 by the same amount of the multiplied culture solution supplied to the reaction vessel 11 (step S4 ; corresponding to a supply process for culture solution for multiplication). As described above, the culture solution for multiplication is supplied in a state that the multiplied culture solution is left in the microorganism pre-cultivation bath 20. Then, cultivation of the microorganism A can be started in the microorganism pre-cultivation bath 20, without supply of new microorganism A strain to the microorganism pre-cultivation bath 20.

It should be noted that supply of the culture solution for multiplication to the microorganism pre-cultivation bath 20 in step S4 can be performed after a prescribed lapse of time, not immediately after supply of the multiplied culture solution to the inside of the reaction vessel 11. Furthermore, in the above-described explanation, though steps S2 to S4 are executed after conducting step S1, it is also possible to execute step S1 after or simultaneously with execution of the respective steps S2 to S4.

When the temperature inside the reaction vessel 11 is in the range from 25 degrees to 45 degrees, the decomposition reaction of organic matter by the microorganism A progresses. However, when multiplication of the microorganism A and the amount of hydrogen produced are considered, it is preferable to adjust the temperature between 30 degrees to 42 degrees. It is also possible to adjust the pressure inside the reaction vessel 11 to be a little negative by sucking the inside air with a pump or the like, after charging of the above-described genus Clostridium microorganism (microorganism A). In such a case, the reaction is more promoted. Furthermore, it is possible to adjust the pH inside the reaction vessel 11, and it is desirable to adjust the pH in the range from 4.0 to 8.0 in this case.

As shown in the flow chart in Fig. 2, it is preferable that while the temperature inside the reaction vessel 11 is controlled to be a prescribed temperature, the fin 15 is rotated inside the reaction vessel 11 by operating the motor 16 so that agitation of the microorganism A and the organic matter is conducted (step S5). By this process, the decomposition reaction of the organic matter progresses uniformly in the reaction vessel 11, and a partial supersaturated state would not be created even when hydrogen is generated and accumulated locally. In other words, it is possible to satisfactorily progress the decomposition reaction over all the organic matter.

Hydrogen produced in the reaction by the decomposition of the organic matter is discharged from the hydrogen discharge pipeline 24 (step S6), and stored in a hydrogen storage unit provided outside such as a hydrogen occlusion alloy, a gas cylinder, or the like. It is also possible to introduce the hydrogen into a combustion chamber immediately to burn it as energy. Furthermore, it is also possible to supply hydrogen to a fuel cell to apply it to power generation.

As also shown in the flow chart in Fig. 2, it is adoptable that the monitor 17 detects whether or not the decomposition reaction to generate hydrogen is completed during decomposition of the organic matter and generation of hydrogen (step S7). In this case, the decomposition reaction of the organic matter is not finished. When the decomposition reaction is not completed and is still continued (No in step S7), the process is returned to step S5, and mix/agitation is continued.

When the monitor 17 determines completion of the decomposition reaction (Yes in step S7), the monitor 17 then detects whether or not the decomposition progresses until the organic matter passes away (in other words, whether or not the decomposition of the organic matter has finished) (step S8). If the decomposition of the organic matter is not completed (No in step S8), it indicates the shortage of the microorganism A necessary for the decomposition of the organic matter. Accordingly, the microorganism A is charged (step S9). After charging the microorganism in step S9, the process is returned to step S5 to continue mix/agitation.

When the monitor 17 determines that the decomposition of the organic matter comes to end in step S8 (in the case of Yes), the decomposition reaction of the organic matter is finished. In other words, when the monitor 17 detects the completion of the decomposition, agitation movement of the fin 15 is stopped. The completion of the reaction is notified outside, for instance, by a buzzer, a lamp or a display means.

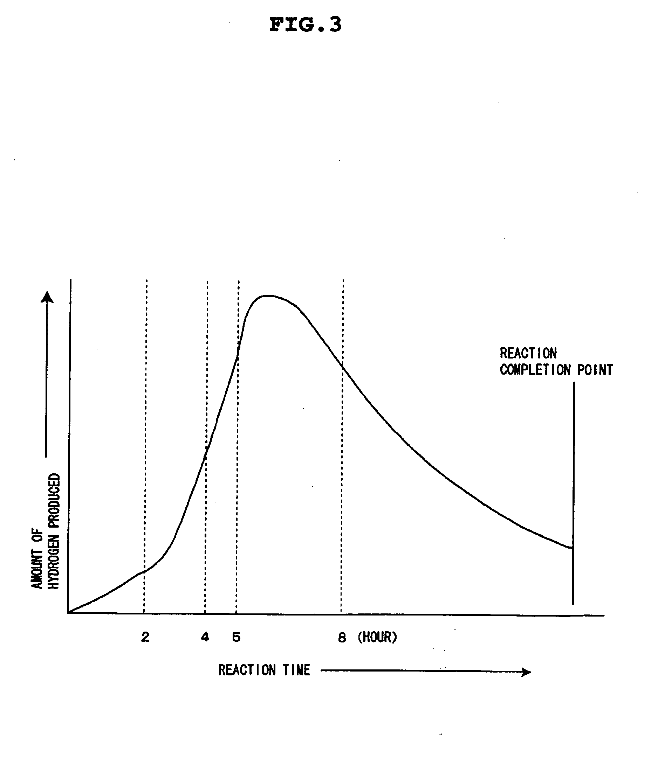

When the decomposition reaction of the organic matter inside the reaction vessel 11 is finished (when it reaches the point of reaction completion shown in Fig. 3), the organic matter left inside the reaction vessel 11 without being decomposed (primary discharge) are discharged (step S10). As above, the process to generate hydrogen is completed.

When the decomposition reaction of the organic matter is continued, the above steps from the above-described step S1 are similarly repeated. Even in such a case, multiplied culture solution is still left inside the microorganism pre-cultivation bath 20 as described above. By arranging in this manner, the microorganism A can be multiplied repeatedly by only supplying the culture solution for multiplication into the inside of the microorganism pre-cultivation bath 20, without supplying the microorganism A into the microorganism pre-cultivation bath 20. Soon after the multiplied culture solution which comes to contain a plenty of new microorganisms A is supplied into the inside of the reaction vessel 11, a new decomposition reaction can be immediately started.

In the above-described method of producing hydrogen, the multiplied culture solution can be charged inside the reaction vessel 11 at any time as necessary. The culture solution for multiplication in which the multiplication has not been sufficiently conducted yet inside the microorganism pre-cultivation bath 20 may be charged inside the reaction vessel 11 at any time as necessary. By conducting in this manner, the decomposition reaction of the organic matter can be further promoted.

An example of the relation between reaction time and amount of generated hydrogen, which is obtained as a knowledge, is shown in Fig. 3. The abscissa axis in Fig. 3 is a reaction time, and the longitudinal axis is the amount of hydrogen produced. Though different according to the organic material selected, the amount of generated hydrogen rapidly increases within about two to four hours after the start of reaction, the amount of generated hydrogen reaches its zenith in about 5 to 8 hours, and then, gradually decreases.

From this relation, it is possible to automatically stop the agitation movement of the fin 15 at the reaction completion time (point of reaction completion), at which the amount of generated hydrogen is expected to decrease to a certain criterion, by detecting the kind and amount of charged materials by the monitor 17 or by previously setting the kind and amount. When it reaches the point of reaction completion, the decomposition reaction of the organic matter for hydrogen production is judged to have reached the point of reaction completion. The organic matter existing inside the reaction vessel 11 at present is discharged to complete the decomposition reaction for hydrogen production using this organic matter.

According to the hydrogen producing apparatus 10 having such a structure, and the method of producing hydrogen, after the microorganism A is sufficiently cultivated in the microorganism pre-cultivation bath 20, the culture solution is supplied to the reaction vessel 11. Since the microorganism A is supplied after the microorganism A is sufficiently cultivated in the microorganism pre-cultivation bath 20 as described above, it becomes unnecessary to charge the strain into the reaction vessel 11 every time when the microorganism A is supplied. Accordingly, once the strain is purchased, the strain can be repeatedly used again and again. Therefore, the decomposition reaction of the organic matter can be economically performed.

Especially, with regard to the microorganism pre-cultivation bath 20, when the multiplied culture solution is supplied inside the reaction vessel 11, the monitor 17 controls amount supplied so as to leave a certain amount of the multiplied culture solution in the microorganism pre-cultivation bath 20. Accordingly, by supplying a new culture solution for multiplication into inside the microorganism pre-cultivation bath 20 from the culture solution tank 21, the cultivation of the microorganism A can be started at once. Therefore, cultivation of the microorganism A can be repeatedly conducted over and over. In addition, in preparation for charging the next organic matter, it is possible to plan a sufficient preparation for new charge by multiplication of the microorganism A.

Furthermore, by providing the microorganism pre-cultivation bath 20, it is possible to supply the multiplied culture solution in which the multiplication of the microorganism A is sufficiently conducted, or the culture solution for multiplication in a state of containing a plenty of the microorganism A, into the inside of the reaction vessel 11 as necessary at any time even in a middle stage of the decomposition reaction of the organic matter. Accordingly, it becomes possible to establish the optimization of the decomposition reaction of the organic matter corresponding to the circumstances of progress of the decomposition reaction of the organic materials, or an environmental variation, thereby making it possible to increase the amount of hydrogen produced per unit time.

The monitor 17 detects the completion of reaction of the organic matter based on the time of reaction from the start of the decomposition reaction of the organic material by the microorganism A, temperatures during progress of the reaction, and the detection result of pH. Accordingly, the monitor 17 always keeps track of progress of the decomposition reaction of the organic matter.

When the decomposition reaction of the organic matter inside the reaction vessel 11 is determined to have completed based on the obtained information of the decomposition reaction progress, it is possible to inform that it is time to charge new organic matter by a notifying means such as a buzzer or a lamp for example. When new organic matter is charged according to this information, and the multiplied culture solution is supplied based on the control by the monitor 17, it is possible to start the decomposition reaction of the new organic matter for hydrogen production immediately.

Further, in the inside of the microorganism pre-cultivation bath 20, it is possible to adjust the culture solution for multiplication or the multiplied culture solution at the temperature suited for the multiplication using the temperature adjusting means, and to keep this temperature after the adjustment. Thus, the microorganism A is multiplied faster in the culture solution for multiplication by adjusting the culture solution for multiplication or the multiplied culture solution at an appropriate temperature, and keeping it at the adjusted state. In the multiplied culture solution in which multiplication has already been conducted, it is possible to keep the state that the multiplication of the microorganism A is being conducted most suitably.

It is also possible to supply the multiplied culture solution in the best suited state for decomposition of the organic matter to the reaction vessel 11 by performing a temperature adjustment in this manner. Accordingly, it becomes possible to increase the amount of hydrogen produced per unit time.

The fin 15 is provided further inside the reaction vessel 11. The fin 15 can promote decomposition reaction of the organic matter by performing agitation of the organic matter inside the reaction vessel 11. Accordingly, it becomes possible to increase the amount of hydrogen produced per unit time.

### (Third Embodiment)

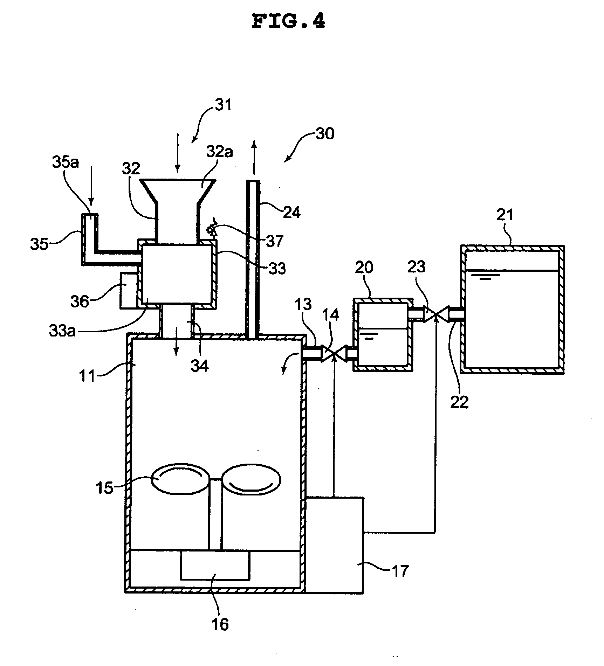

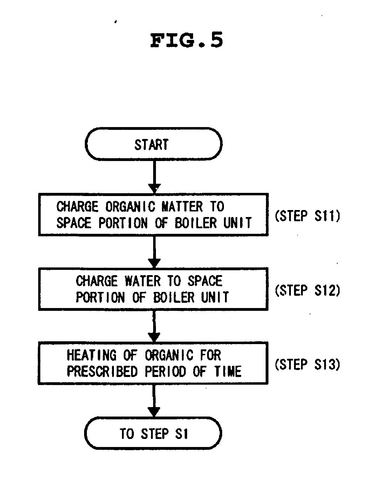

The third embodiment of the present invention will be explained based on Figs 4 and 5. Note that in the present embodiment, the same structure as described in the above second embodiment will be explained using the same numerals and symbols.

In the present embodiment, a sterilization mechanism 31 is added to the hydrogen producing apparatus 10 described in the above-described second embodiment. A hydrogen producing apparatus 30 including the sterilization mechanism 31 will be described in detail hereinafter. Note that the sterilization mechanism 31 described as follows includes a piping member 32, a boiler unit 33, a water intake pipe 35, a heating mechanism 36, and a pressure adjusting mechanism. However, in order to be the sterilization mechanism 31, it is satisfactory so far as it is provided with at least the boiler unit 33 to perform sterilization treatment by boiling the organic matter.

As shown in Fig. 4, the sterilization mechanism 31 is provided at the middle of the piping member 12 in the above-described second embodiment. The sterilization mechanism 31 is provided with a material charging port 32a similar to that described in the second embodiment. The material charging port 32a is formed on one end (upper end) of the piping member 32 with the mouth open.

The other end of the piping member 32 is connected to the boiler unit 33. The boiler unit 33 includes a space portion 33a capable of storing a prescribed amount of the organic matter charged inside thereof. Accordingly, the organic matter charged via the material charging port 32 temporarily exist in the space portion 33a of the boiler unit 33.

The boiler unit 33 is connected to the reaction vessel 11 via a connecting pipe 34. The connecting pipe 34 is provided with an open/close lid (not shown) at an aperture on the boiler unit 33 side. It becomes possible to supply the organic matter which have completed the sterilization treatment to the reaction vessel 11 side by opening the open/close lid. It is also possible to charge the organic matter in a state of closing the open/close lid, and conduct boiling of the organic matter while pouring water from a water charging port 35a to be described later.

The other end of the water intake pipe 35 as a liquid pouring means is connected to the boiler unit 33. One end side of the water intake pipe 35 serves as the water charging port 35a so as to supply a sufficient amount of water to the space portion 33a of the boiler unit 33. The boiler unit 33 is provided with a heating mechanism 36. The heating mechanism 36 is a mechanism to heat (boil) the organic matter and water in a state that the organic matter and water are supplied to the space portion 33a. The sterilization treatment of the organic matter existing in the space portion 33a is conducted by heating in this manner.

The water intake pipe 35 (water charging port 35a) is not necessarily provided, and it is possible to heat only the boiler unit 33 with the heating mechanism 36. It is also possible to supply boiling water, other liquid, water vapor, or the like instead of water.

The boiler unit 33 is provided with a pressure adjusting mechanism 37. The pressure adjusting mechanism 37 is for keeping the pressure of the space portion at an appropriate pressure when heating the space portion by the heating mechanism 36. It is possible to adjust the inside of the space portion at temperatures and pressures suitable for the sterilization treatment by provision of the pressure adjusting mechanism 37 together with the heating mechanism 36.

Hereinafter, a method of producing hydrogen by using the hydrogen producing apparatus 30 as described above will be explained using Fig. 5.

In the present embodiment, the sterilization treatment of the organic matter is conducted before charging the organic matter inside the reaction vessel 11 (corresponding to the sterilization treatment process). When the sterilization treatment is conducted, the organic matter is charged to the space portion 33a of the boiler unit 33 from the material charging port 32a (step S 11). After charging the organic matter, a prescribed amount of water is poured into the space portion 33a from the water charging port 35 (step S12). Accordingly, the space portion 33a is in a state of mixing the organic matter with water. It is also possible to conduct step S12 before performing step S 11 or simultaneously with step S 11.

The heating mechanism 36 is operated in this state to heat the organic matter in a mixture state with water existing in the space portion 33a (step S 13). In this case, it is preferable that the heating mechanism 36 heats the organic matter to about 80 degrees. However, the temperature of heating is not limited to about 80 degrees, but it may be, for instance, 80 degrees or above, or a temperature in the range between about 65 degrees and about 80 degrees, so far as it is the temperature to be able to perform the sterilization treatment favorably.

As described above, accompanied by heating to about 80 degrees being a favorable temperature, water is evaporated into vapor, and the pressure inside the space portion 33a is increased. At the time of the pressure increase, the pressure adjusting mechanism 37 is operated to adjust pressure in the space portion 33a to be an appropriate pressure. It is preferable to adjust to 1.2 to 1.3 atmospheric pressures as the appropriate pressure. However, the pressure in the space portion 33a is not limited to 1.2 to 1.3 atm., but may be more than 1.2 to 1.3 atm., or less than the range.

The conditions at such a temperature and a pressure are maintained for a prescribed period. It is preferable as the prescribed period to be about 5 to about 20 minutes. As above, when the organic matter existing in the space portion 33a are boiled for the prescribed period under conditions at preferable temperatures and pressures, saprophytic bacteria or the like contained in the organic matter is extinct, which can be taken as execution of the sterilization treatment.

After such a sterilization treatment is finished, the organic matter is supplied inside the reaction vessel 11 via the connecting pipe 34. Note that the supply of the organic matter corresponds to step S 1 in Fig. 2.

The treatment steps after supply of the organic matter to the reaction vessel 11 is similar to the steps (refer to Fig. 2) described in the second embodiment. That is, also in the present embodiment, when the microorganism A is newly supplied to the reaction vessel 11, the multiplied culture solution is kept remained inside the microorganism pre-cultivation bath 20. By this arrangement, it is possible to cultivate the microorganism A repeatedly only by supplying the culture solution for multiplication to the inside of the microorganism pre-cultivation bath 20 without supplying the microorganism A to the microorganism pre-cultivation bath 20.

Next, the multiplied culture solution in a state of containing a plenty of the microorganism A is supplied inside the reaction vessel 11, thereby starting the decomposition reaction of the organic matter to generate hydrogen.

According to the hydrogen producing apparatus 30 having such a structure, since the organic matter is supplied to the reaction vessel 11 after the sterilization treatment, the organic matter supplied to the reaction vessel 11 can be in a state having no other saprophytic bacteria attached. When the multiplied culture solution is supplied in such a state, the microorganism A (genus Clostridium microorganism in the present embodiment) contained in the multiplied culture solution to produce hydrogen can efficiently decompose the organic matter without being affected by other bacteria.

Since the microorganism A for hydrogen production can efficiently decompose the organic matter, it is possible to increase the amount of hydrogen produced per unit time. By conducting the sterilization treatment by boiling as in the present embodiment, the space portion 33a of the boiler unit 33 also gets a state that the sterilization treatment is conducted. Accordingly, even when the organic matter is repeatedly supplied to the reaction vessel 11, since saprophytic bacteria do not multiply in the space portion 33a of the boiler unit 33, cleanliness can be maintained.

The sterilization mechanism 31 includes the boiler unit 33 into which the organic matter is charged, and the heating mechanism 36 to heat the organic matter charged into the boiler unit 33. Accordingly, the organic matter charged into the boiler unit 33 is heated by the heating mechanism 36, and saprophytic bacteria contained in the organic matter can be extinct. In other words, when saprophytic bacteria is heated (boiled) to 80 degrees or higher, they will be almost extinct. Accordingly, by this structure, it is possible to reliably perform sterilization treatment. In addition, it is possible to increase the amount of hydrogen generated per unit time from the organic matter charged inside the reaction vessel 11 by performing reliable sterilization treatment.

Furthermore, the sterilization mechanism 31 is provided with the water charging port 35a to pour water into the boiler unit 33. Accordingly, when the sterilization treatment is conducted, liquid such as water or the like is charged together with the organic matter into the inside of the boiler unit 33. In this state, by operating the heating mechanism 31, high fluidity liquid is heated inside the boiler unit 33. Thereby, the organic matter can be heated uniformly, so that reliable sterilization treatment of the organic matter can be performed. In addition, it is possible to increase the amount of hydrogen generated per unit time from the organic matter charged inside the reaction vessel 11 by performing reliable sterilization treatment.

Though the present embodiment has a structure to have the sterilization mechanism 31 in addition to a mechanism of supplying the microorganism A repeatedly (the microorganism pre-cultivation bath 20 and the culture solution tank 21), which is described in the second embodiment, a structure to provide with the sterilization 31 in a state of elimination of such a mechanism for supplying the microorganism A repeatedly may be adopted. In this case also, it is possible to perform such a function as increase of the amount of hydrogen produced and the maintenance of cleanness.

### (Fourth Embodiment)

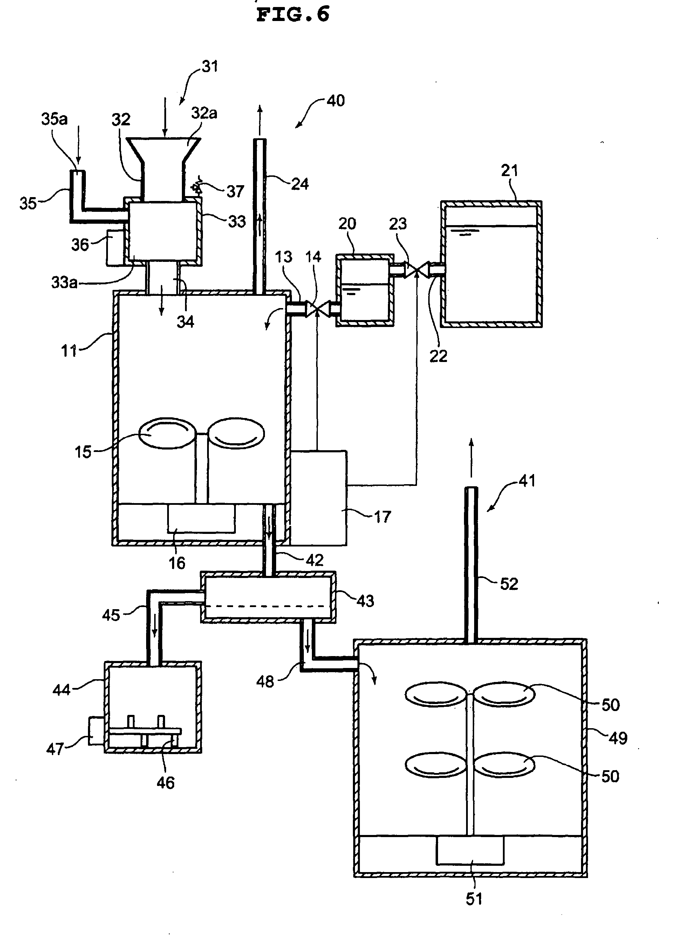

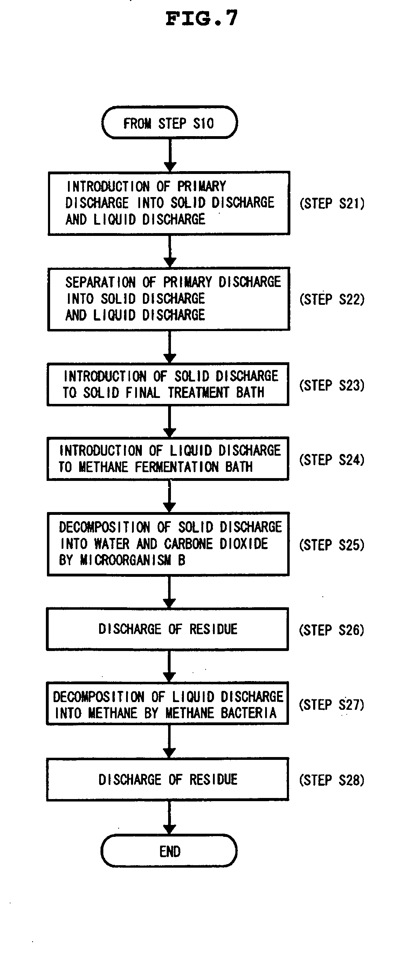

Hereinafter, the fourth embodiment of the present invention will be explained based on Figs. 6 and 7. The present embodiment relates to a hydrogen producing apparatus 40 provided with a methane producing mechanism 41 in addition to the structure described in the above described third embodiment. The same structures as described in the second and third embodiments are explained using the same numerals and symbols. In the present embodiment, a structure provided with a solid final treatment bath 44 for treating a solid discharge other than the methane producing mechanism 41 will be explained as will be described later.

As shown in Fig. 6, on the bottom side of the reaction vessel 11, a solid-liquid separation bath 43 as a solid-liquid separation means is connected via a discharge piping 42. The solid-liquid separation bath 43 is for separating a primary discharge after completion of decomposition reaction in the reaction vessel 11 into a solid discharge and a liquid discharge. In other words, in the reaction vessel 11, when decomposition reaction of the organic matter progresses, hydrogen is generated, and at the same time, water and other gases (carbon dioxide or the like) are generated. Water poured from the water charging port 35a provided at the sterilization mechanism 31 described in the above described third embodiment exists also inside the reaction vessel 11.

Accordingly, the primary discharge after completion of hydrogen production in a state of mixing with water (liquid) is separated in the solid-liquid separation bath 43 into a solid and liquid. It should be noted that a method for separating a solid and liquid, various methods such as separation by a filter or the like can be used.

The solid final treatment bath 44 as a means for final treatment of a solid is connected to the solid-liquid separation bath 43 via piping for a solid. The solid final treatment bath 44 is for separating a solid discharge after conducting solid-liquid separation of the primary discharge into water and carbon dioxide. Such a solid discharge is decomposed into water and carbon dioxide using a prescribed microorganism in the solid final treatment bath 44.

As a microorganism to be used, the following disclosed in the patent application from Japanese Patent Application No. 2001-167101 are favorable. They are Bacillus amyloliquefaciens 148 (Acceptance No.; FERM P-18349), Bacillus amyloliquefaciens 2414 (Acceptance No.; FERM P-18347), Bacillus subtilis 237 (Acceptance No.; FERM P-18350), Strain 4. Bacillus licheniformis 136 (Acceptance No.; FERM P-18346), and Strain 5. Bacillus licheniformis 2530 (Acceptance No.; FERM P-18348).

When such a microorganism is used, it is possible to satisfactorily decompose a solid discharge into water and carbon dioxide. However, as the microorganism for treatment used in the solid final treatment bath 44, it is not limited to the above described strains 1 to 5, and any microorganism can be used so far as it can decompose the above described solid discharge into water and carbon dioxide favorably. Hereinafter, a microorganism to decompose into water and carbon dioxide including these strains 1 to 5 will be described as a microorganism B in the following explanation.

The solid final treatment bath 44 is provided with a fin 46 inside thereof as a third agitating means. The solid discharge in the solid final treatment bath 44 is agitated with the fin 46. Thereby, a decomposition reaction into water and carbon dioxide etc. of the solid discharge is promoted. Note that a motor 47 is provided to rotationally drive the fin 46 in the solid final treatment bath 44.

Furthermore, a methane fermentation bath 49 as a methane fermenting means is connected to the solid-liquid separation bath 43 other than the solid final treatment bath 44 via liquid piping 48. The methane fermentation bath 49 is for producing methane from a liquid discharge after solid-liquid separation of the primary discharge is performed. In order to generate methane, the methane fermentation bath 49 takes a state of charging methane bacteria in advance into the inside of the methane fermentation bath 49. Thereby, the liquid discharge is decomposed by the methane bacteria, and methane gas is able to be generated. Note that methane bacteria explained in the above-described first embodiment can be used as the methane bacteria.

A fin 50 is provided as a second agitating means inside the methane fermentation bath 49. The fin 50 is rotationally driven by the motor 51 provided in the methane fermentation bath 49. Thereby agitating inside the methane fermentation bath 49, it becomes possible to realize promotion of the reaction for methane fermentation.

One end of a methane discharge pipeline 52 is connected to the upper end of the methane fermentation bath 49. The other end of the methane discharge pipeline 52 is connected to a methane storage unit (not shown). Accordingly, it becomes possible to withdraw methane generated inside the methane fermentation bath 49.

A method of producing hydrogen using the above-described hydrogen producing apparatus 40 will be described based on Fig. 7.

A flow shown in Fig. 7 shows the steps of methane fermentation treatment for producing methane, and conducting final treatment to decompose it into water and carbon dioxide, for the primary discharge created after producing hydrogen from the organic matter in the reaction vessel 11. In other words, the flow shown in Fig. 7 is that showing a treatment step conducted after step S 10 in the above-described second embodiment.

The primary discharge is introduced at first into the solid-liquid separation bath 43 via the discharge piping 42 (step S21). In the solid-liquid separation bath 43, the primary discharge is separated into a solid discharge and a liquid discharge by, for instance, passage through a filter (step S22; corresponds to a solid-liquid separation process). The solid discharge out of these two discharges is introduced to the solid final treatment bath 44 via piping for solid 45 (step S23). The liquid discharge is introduced to the methane fermentation bath 49 via the piping 48 for liquid (step S24).

The microorganism B is beforehand supplied to the solid final treatment bath 45 out of the two. Accordingly, the solid discharge is decomposed into water and carbon dioxide by the microorganism B (step S25; corresponds to a solid final treatment process). Note that the solid discharge inside the solid final treatment bath 45 is agitated by drive of the fin 46. A decomposition reaction of the solid discharge is promoted by this agitation.

When the decomposition reaction of the solid discharge is progressed in the solid final treatment bath 44, the solid discharge almost disappears as the decomposition of the solid discharge into water and carbon dioxide progresses, and only a portion thereof such as a fish born is left. The remained residue is discharged as a secondary discharge (step S26). As described above, a decomposition reaction for the final treatment of the solid discharge is completed.

The methane bacteria supplied in advance to the methane fermentation bath 49. Accordingly, it is possible to decompose the liquid discharge with a methane bacteria to produce methane (step S27; corresponds to a methane fermentation process). Note that the liquid discharge inside the methane fermentation bath 49 is agitated when methane is generated. The decomposition treatment inside the solid final treatment bath 44 and the methane fermentation bath 49 is conducted for a prescribed time, and when both decomposition reactions are considered to have progressed sufficiently, the reactions are suspended. A residue not decomposed in the methane fermentation bath 49 is discharged as a secondary discharge. As above, the decomposition reaction for methane production is completed.

In the above explanation, the respective steps S23 to S28 are designed to be conducted according to this sequence. However, so far as the conditions that steps S25 and S26 are executed in sequence after step S23 and steps S27 and S28 are executed in sequence after step S24 are fulfilled, execution of the respective steps S23 to S28 can be performed in any order.

According to the hydrogen producing apparatus 40 having such a structure, it is possible to produce methane by using the primary discharge created at the time of hydrogen production. In other words, the primary discharge after completion of the decomposition reaction for hydrogen production is separated into a solid discharge and a liquid discharge by the solid-liquid separation bath 43. When the liquid discharge is used out of these two kinds of discharges, methane can be produced by the function of the methane bacteria inside the methane fermentation bath 49. Accordingly, it becomes possible to produce fuel more efficiently by using the organic matter so that effective utilization of the organic matter can be realized.

As above, according to the hydrogen producing apparatus 40 in the present embodiment, not only hydrogen can be produced from the organic matter in the inside of the reaction vessel 11, but also methane can be produced in the inside of the methane fermentation bath 49.

Furthermore, in the solid final treatment bath 44, it is possible to decompose nearly completely the solid discharge after separating the primary discharge into water and carbon dioxide favorably. Thus, it is possible to treat the cast solid discharge in an ideal state with generation of little discharge (garbage) by providing the solid final treatment bath 44.

Since the hydrogen producing apparatus 40 is provided with the solid final treatment 44 in addition to the above-described methane fermentation bath 49, it is possible to decompose the solid discharge into water and carbon dioxide by the microorganism B of the above-described strains 1 to 5. By this decomposition, little discharge (garbage) is produced from the solid final treatment bath 44. Accordingly; the hydrogen producing apparatus 40 according to the present embodiment, is ideal as a garbage treatment apparatus.

The above-described microorganism B which decomposes the organic matter into water and carbon dioxide can be repeatedly used inside the solid final treatment bath 44. Accordingly, there is no need to put a new microorganism B at the time of treatment of solid discharge, and little cost is required.

Both the solid final treatment bath 44 and the methane fermentation bath 49 are provided with the fin 46 and the fin 50 according to the present embodiment. Accordingly, decomposition reaction of the solid discharge in the solid final treatment bath 44, and methane fermentation inside the methane fermentation bath 49 can be promoted by driving these fins 46 and 50, thereby enabling efficient final treatment of the solid discharge and efficient methane fermentation to be conducted.

Though the respective embodiments 1 to 4 of the present invention are explained as above, various modifications of the present invention are also possible to be put into practice.

The above-described first embodiment explains a method of producing hydrogen using biomass containing food related waste and/or living related waste. However, a raw material for producing hydrogen is not limited to biomass containing such food related waste and/or living related waste as the raw material for producing hydrogen. It is possible to generate hydrogen using various biomass such as, for instance, agricultural resources using energy crops other than waste, forestry resources using energy plants, livestock industry resources, marine products industry resources, and the like.

Furthermore, in the above-described first embodiment, it is possible to provide various means described in the above-described second to fourth embodiments such as first agitating means, controlling means, culture solution storage means, microorganism pre-cultivating means, sterilization mechanism, liquid-solid separating means, second agitating means, solid final treatment means, third agitating means, fluid casting means, and the like.

Though the fin 15 is provided inside the reaction vessel 11 in the above-described second to fourth embodiments, it is possible to adopt a structure which eliminates the fin 15. Similarly, though in the fourth embodiment, the fin 46 is provided in the solid final treatment bath 44, and the fin 50 is provided in the methane fermentation bath 49 respectively, it is possible to adopt a structure to eliminate the fins 46 and 50 respectively.

In the above-described second to fourth embodiments, completion of the decomposition reaction of the reaction vessel 11 is detected based on reaction time from decomposition start, temperature during progress of reaction, detection result of pH. However, the completion of the decomposition reaction is not limited to this, other methods can be used to detect it. Further, when, for instance, a prescribed amount of the organic matter is put inside the reaction vessel 11, the reaction can be determined to have finished after a predetermined time elapses.

Though provision of a temperature adjusting means in the microorganism pre-cultivation bath 20 is explained in the above-described second to fourth embodiments, it is possible to adopt a structure to eliminate such a temperature adjusting means, when, for instance, the external atmosphere is kept at a suitable temperature.

In the above-described second to fourth embodiments, hydrogen may be occluded using carbon nanotube as described in the first embodiment. Similarly, in the above-described second to fourth embodiments, it is also possible to store methane by means of compression process as described in the first embodiment.

In the third and fourth embodiments, though the sterilization mechanism 31 is explained to include the boiler unit 33 and the heating mechanism 36, it is also possible to adopt a structure to conduct sterilization treatment of the organic matter by means of, for instance, irradiating microwave beams on the organic matter instead of the above-described structure. Additionally, in the above-described third and fourth embodiments, though provision of the water charging port 35 to the sterilization mechanism 31 is explained, it is possible to adopt a structure to simply heat the organic matter instead of boiling them.

In the fourth embodiment, a structure provided with the methane producing mechanism 41 in addition to the sterilization mechanism 31 described in the above described third embodiment is explained. However, it is possible to adopt a structure provided with the methane producing mechanism 41 without provision of the sterilization mechanism 31. It is also possible to adopt a structure provided with the methane producing mechanism, but to omit the solid final treatment bath 44 (solid final treatment means). It is again also possible to adopt a structure to be provided with only the solid final treatment bath 44 (solid final treatment means) without provision of the methane producing mechanism 41.

Further, in the above described fourth embodiment, it is possible to adopt a structure to adjust the inside of the solid final treatment bath 44 or the methane fermentation bath 49 to suitable pressure and temperature for decomposition reaction or fermentation.

Furthermore, in the above-described respective embodiments, though the microorganism pre-cultivation bath 20 is described as a microorganism pre-cultivating means, the microorganism pre-cultivating means is not limited to the microorganism pre-cultivation bath 20, and any structure can be used so far as it can pre-cultivate the microorganism A (for instance, microorganism pre-cultivation tank or the like) in a state of supplying a culture solution for multiplication. In addition, for the culture solution tank 21 as a culture solution storing means, it is possible to use any structure so far as it can store the culture solution for multiplication (for instance, a culture solution storage bath or the like).

### Industrial Availability

The method of biomass processing of the present invention can be applied in an energy field such as a fuel cell or the like.

## Claims

1. A method of biomass processing, comprising the steps of:
recovering hydrogen by conducting hydrogen fermentation process of biomass with hydrogen producing bacteria containing at least one out of Clostridium beijerinkii AM21B strain, Clostridium sp. No. 2 strain, and Clostridium sp. X53 strain; and
recovering methane by conducting methane fermentation treatment of a fermentation liquid generated after the hydrogen fermentation process with methane bacteria,
wherein said the fermentation liquid contains must contain any organic acid.

2. The method of biomass processing according to claim 1, wherein
said biomass contains food related waste and/or living related waste.

3. The method of biomass processing according to claim 1 or claim 2, wherein
said hydrogen recovered by said hydrogen fermentation process is formed in a liquid state by compression process.

4. The method of biomass processing according to claim 1 or claim 2, wherein
said hydrogen recovered by said hydrogen fermentation process is occluded by carbon nanotube.

5. The method of biomass processing according to claim 1 or 2, wherein
said hydrogen recovered by said hydrogen fermentation process is occluded by a hydrogen occlusion alloy.

6. The method of biomass processing according to any one of claims 1 to 5, wherein
said methane recovered by said methane fermentation treatment is formed in a liquid form by compression process.

7. The method of biomass processing according to any one of claims 1 to 6, wherein said methane fermentation treatment is conducted using at least one of bacteria belonging to genus Methanobacterium, genus Methanococcus, genus Methanosarcina, genus Methanosaeta, and genus Methanohalophillus as said methane bacteria.
